# EUROPEAN PATENT APPLICATION

(11) **EP 4 748 305 A1**
(43) Date of publication of application: **27.05.2026**
(21) Application number: 25189302.0
(22) Date of filing: 14.07.2025
(51) Int. Cl.: A61B 5/08, A61B 5/11

(54) **MULTI-MODAL BASED SHORTNESS OF BREATH ESTIMATION**

(30) Priority: 31.07.2024 US 202463677925 P; 03.07.2025 US 202519259861
(71) Applicant: Apple Inc., Cupertino, CA 95014 (US)
(72) Inventor: LEZZOUM, Narimene, Cupertino, 95014 (US); ARNEY, Julie A., Cupertino, 95014 (US); WAKUTSU, Lessa, Cupertino, 95014 (US); O'REILLY, Michael, Cupertino, 95014 (US); BURUGULA, Shashank Dhar, Cupertino, 95014 (US); ULLAL, Adeeti V., Cupertino, 95014 (US)
(74) Representative: Barton, Russell Glen

(57) **Abstract**

Multi-modal shortness of breath systems and methods are described. In aspects, one or more devices may be utilized to collect data associated with the user, such as audio data (e.g., speech pattern, breath, etc.) and motion data(e.g., walking, exercising, etc.) that overlaps in time with the audio data. Further, an assessment system may analyze both the audio data and the motion data collected by the one or more devices to provide an overall health and/or fitness metric for the user.

## Description

### BACKGROUND

### RELATED APPLICATIONS

This application claims the benefit of priority of U.S. Provisional Application No. 63/677,925, filed July 31, 2024, which is herein incorporated by reference.

### FIELD

Aspects described herein generally relate to developing machine learning models for health and fitness applications.

### BACKGROUND INFORMATION

Respiration is an important metric for our health, fitness, and wellness. The respiratory effort that a person takes while either engaging in an activity or being sedentary may be an indicator of different health-related issues such as heart and lung diseases.

### SUMMARY

Multi-modal shortness of breath estimation systems, devices and methods are described. In an aspect, an assessment system may include one or more devices that collect data associated with the user (e.g., audio data, motion data, physiological data, environmental data, etc.). The data associated with the user may include audio data (e.g., talk test, speech pattern, etc.), where the assessment system may analyze the audio data to generate a shortness of breath metric. Further, the data associated with the user may include motion data (e.g., walking, etc.) that overlaps in time with the audio data, where the motion data may be analyzed to generate an activity metric. Further still, the assessment system may analyze the shortness of breath metric and the activity metric to generate an overall assessment metric. In some aspects, the data collected by the one or more devices (e.g., audio data, motion data, etc.), as well as the metrics that correspond to such data, may be analyzed by multiple different machine learning models or by the same (e.g., a singular) machine learning model.

### BRIEF DESCRIPTION OF THE DRAWINGS

FIG. 1A is an illustration of one or more devices that may be utilized in accordance with aspects.
FIG. 1B is a schematic illustration of multiple capture devices in accordance with aspects.
FIG. 2A is a schematic illustration of an assessment system that includes a prompt module in accordance with aspects.
FIG. 2B is a schematic illustration of an assessment system that includes a passive model in accordance with aspects.
FIG. 2C is a schematic illustration of an assessment system that includes an example multi-modal subsystem in accordance with aspects.
FIG. 2D is a schematic illustration of an assessment system that includes another example multi-modal subsystem in accordance with aspects.
FIG. 3 is a schematic illustration of a training phase in accordance with aspects.
FIG. 4 is an illustration of an example user interface of an application in accordance with aspects.
FIG. 5 is a flow chart of a method for generating a shortness of breath metric in accordance with aspects.

### DETAILED DESCRIPTION

It has been observed that shortness of breath (also referred to as breathlessness or dyspnea) reflects the respiratory effort that a person takes while either doing an activity or being sedentary. In the fitness space, a talk test can measure the level of intensity the person may experience while engaging in a physical activity by observing changes in speech patterns over the course of the physical activity. In the health space, clinicians may ask patients to undergo a small physical activity (e.g., walking up stairs, etc.) and then rate their level of shortness of breath according to a standard scale (e.g., modified medical research council dyspnea scale, etc.). However, such tests may only be administered intermittently and may be subjective based on the assessments of third parties.

In accordance with aspects, assessment systems and methods provide speech-based shortness of breath estimations for health and fitness applications. In one aspect, one or more devices may be utilized to collect data associated with the user. The data associated with the user may include audio data from a user, where such audio data may include a speech pattern of the user. In some instances, the audio data may include a breathing pattern of the user (e.g., inhale, exhale, etc.). In another aspect, the data associated with the user may include motion data from a user (e.g., walking, running, etc.), where such motion data may overlap in time with the audio data collected by the one or more devices. In some instances, the motion data may include sedentary activity (e.g., sitting at a desk, meditating, etc.). In another aspect, an assessment system may analyze both the audio data and the motion data collected by the one or more devices to provide an overall health and/or fitness metric for the user.

In various aspects, description is made with reference to figures. However, certain aspects may be practiced without one or more of these specific details, or in combination with other known methods and configurations. In the following description, numerous specific details are set forth, such as specific configurations, dimensions and processes, etc., in order to provide a thorough understanding of the aspects. In other instances, well-known machine-learning models and techniques have not been described in particular detail in order to not unnecessarily obscure the aspects. Reference throughout this specification to "one aspect" means that a particular feature, structure, configuration, or characteristic described in connection with the aspect is included in at least one aspect. Thus, the appearances of the phrase "in one aspect" in various places throughout this specification are not necessarily referring to the same aspect. Furthermore, the particular features, structures, configurations, or characteristics may be combined in any suitable manner in one or more aspects.

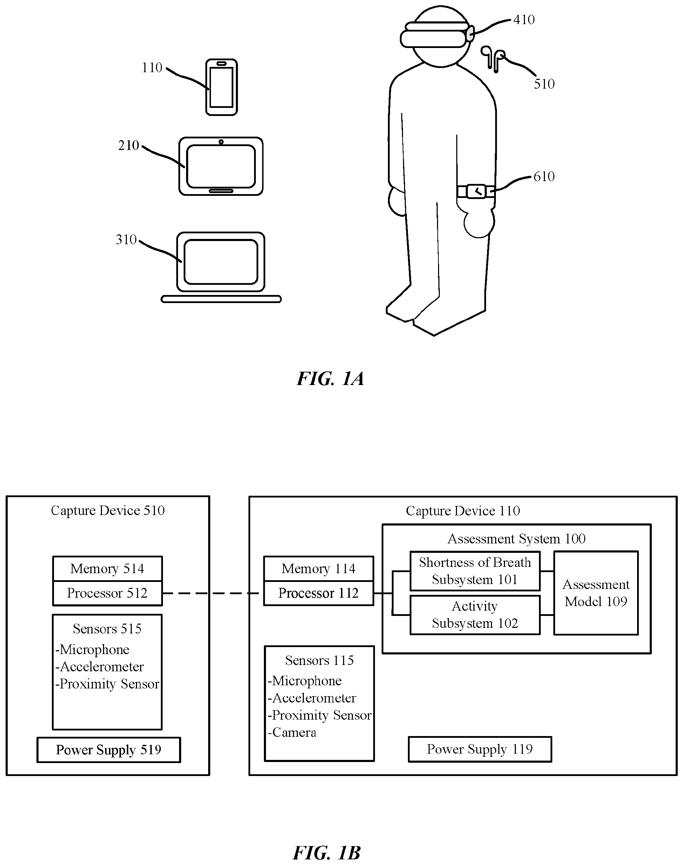

Referring now to FIG. 1A, an illustration of one or more devices that may be utilized to provide a health and/or fitness assessment of a user is shown. In the example of FIG. 1A, a group of one or more devices may include capture device 110 (e.g., smartphone), capture device 210 (e.g., tablet), capture device 310 (e.g., laptop computer, desktop computer, etc.), capture device 410 (e.g., mixed-reality headset, virtual reality headset, etc.), capture device 510 (e.g., earbuds, earphones, headphone, etc.), and capture device 610 (e.g., smartwatch, etc.). It should be noted that the above-described capture devices in the group of the one or more devices in FIG. 1A do not represent an exhaustive list of capture devices and that other types of capture devices are also contemplated. For example, capture devices may include other types of wearable devices (e.g., smart rings, smart clothing, fitness monitors, smart patches, etc.), other types of non-wearable devices (e.g., smart home devices, environmental sensors, voice analysis systems, etc.), medical-grade devices (e.g., pulse oximeters, spirometers, etc.), etc.

Referring now to FIG. 1B, a schematic illustration of multiple capture devices is shown. The one or more devices may collect data associated with the user, where collecting data associated with the user may include acquiring signals from sensors (e.g., waveforms, accelerometer output, gyroscope signals, etc.), receiving processed data from other systems/subsystems, and obtaining information through any acquisition method. For example, the one or more sensors may collect audio data (e.g., speech pattern, breath, etc.) and/or motion data (e.g., walking, exercising, etc.) to provide a health or fitness assessment of the user, where such data may be captured by a singular capture device (e.g., smartphone, etc.) or by multiple capture devices in tandem (e.g., smartphone and earbuds, etc.). A speech pattern of the user may include various characteristics of speech, such as rhythm, tone, pitch, etc. In addition, changes in the speech pattern may be reflected by changes in such speech characteristics, or, for example, in the ability (or inability) to repeat a sentence after engaging in a particular activity (e.g., talk test, etc.). Further, the breath of the user may include respiratory characteristics related to inhaling and exhaling, such the respiratory rate, breathing depth, etc., where changes in the breath may be reflected by changes in such respiratory characteristics.

In further reference to FIG. 1B, each of the one or more devices, respectively, may include processing circuitry (e.g., central processing unit, etc.), memory (e.g., read-only memory, random access memory, etc.), sensors (e.g., microphones, accelerometers, proximity sensors, cameras, optical sensors, physiological sensors, environmental sensors, temperature sensors, gyroscopes, etc.) and power supplies contained within a housing. In addition, processing circuitry may perform audio and motion analyses, respectively, on the audio data and motion data collected by the one or more devices, such as removing noise, adding annotations/labels, converting signals into corresponding visual representations (e.g., waveforms, spectrum plots, spectrograms, etc.), etc., where the corresponding visual representations may then be further analyzed by the machine learning models of assessment system 100. Further, the processing circuitry may also include networking technology to enable wireless communication (e.g., Bluetooth, Wi-Fi, etc.) between the one or more devices and/or an external computer system (e.g., server, cloud network, etc.). In the example of FIG. 1B, capture device 110 (e.g., smartphone) may include processing circuitry 112 coupled to memory 114, and power supply 119, where processing circuitry 112 may execute a set of instructions stored on memory 114 to collect or capture data from sensors 115, where such components may be contained within a housing. In addition, capture device 510 (e.g., earbuds) may include processing circuitry 512 coupled to memory 514, and power supply 519, where processing circuitry 512 may execute a set of instructions stored on memory 514 to collect or capture data from sensors 515, where such components may be contained within a housing.

In further reference to FIG. 1B, each of the one or more devices may include assessment system 100 to perform operations related to a health and/or fitness assessment of the user. Further, assessment system 100 may include multiple subsystems (e.g., shortness of breath subsystem 101, activity subsystem 102, etc.) and machine learning models (e.g., assessment model 109, etc.). Persons of ordinary skill in the art will appreciate that the machine leaning models of assessment system 100 can be any suitable machine learning models that are well-known or widely available such as regression techniques, classification techniques, decision trees, neural networks, and deep learning networks. For instance, assessment system 100 can include Artificial Neural Network (ANN), Convolutional Neural Network (CNN), Recurrent Neural Network (RNN), Generative Adversarial Network (GAN), Reinforcement Learning Model (RLM), and/or Transformer-Based Models (e.g., Bidirectional Encoder Representations from Transformers (BERT), Generative Pre-trained Transformer (GPT), and/or a multi-modal large language model). Additionally, or alternatively, persons of ordinary skill in the art will appreciate that assessment system 100 can be any suitable non-learning systems such as rule-based systems, heuristics, decision trees, knowledge-based systems and expert systems.

It has been observed that machine learning models that include sensitive data, such as health and fitness related data, may perform operations on-device so the data does not leave the device and thereby become more susceptible to breaches. As such, assessment system 100 may be located on any or all of the one or more devices so that the machine learning models of assessment system 100 may be performed on-device. For example, as illustrated in FIG. 1B, assessment system 100 is located on capture device 110. It has also been observed that some devices may be restricted with respect to storage, memory, compute resources, and power consumption limitations so that such devices may not be suitable for executing machine learning models. As such, in aspects, assessment system 100 may be located in whole or in part on any of the one or more devices so that the multiple subsystems (e.g., shortness of breath subsystem 101, activity subsystem 102, etc.) or multiple machine learning models (e.g., assessment model 109, etc.) of assessment system 100 may be located on multiple different devices that may be communicatively linked. In this way, multiple devices may jointly perform the operations of assessment system 100 so as to overcome any device restrictions with respect to storage, memory, compute resources, power consumption limitations, etc.

In one aspect, at least one device may serve as a capture device to collect data, whereas at least one other device may serve as a companion device to execute the machine learning models for the collected data. For example, as illustrated in FIG. 1B, capture device 510 (e.g., earbud device) may collect data from sensors 515, where processing circuitry 512 may cause such data to be wirelessly transmitted (e.g., Bluetooth, etc.) to processing circuitry 112 of capture device 110 so that capture device 110 may serve as a companion device (in addition to being a capture device itself) to execute the machine learning models of assessment system 100. In another aspect, at least one device may serve as a capture device to collect data and execute at least a first machine learning model on the collected data, whereas at least one other device may serve as a companion device to execute at least a second machine learning model on the collected data. For example, one subsystem of assessment system 100 may be located on a first device, such as shortness of breath subsystem 101 that may be located on capture device 510, whereas another subsystem of assessment system 100 may be located on a second device, such as activity subsystem 102 that may be located on capture device 110. In another aspect, assessment system 100 may be located in whole or in part on an external computer system (e.g., physical server, virtual server, etc.) where the operations of assessment system 100 may be performed remotely. Further, it should be noted that in such aspects, any of the one or more devices may serve as either a capture device, a companion device or both in connection with the same health and/or fitness metric determination.

In addition, multiple different devices of the one or more devices may collect data associated with the user. In such instances, assessment system 100 may select data from one, some or all of the one or more devices based on the characteristics of the audio data. In one aspect, assessment system 100 may select a primary capture device from the one or more devices to collect data associated with the user. In one example, assessment system 100 may select the audio data from the capture device closest to the user. For example, where capture device 110 (e.g., smartphone) and capture device 510 (e.g., earbuds) collect audio data from a user, assessment system 100 may select capture device 510 where sensors 515 (e.g., proximity sensor) indicate that capture device 510 is near the user's face (e.g., in-ear) and sensors 115 (e.g., proximity sensor) indicate that capture device 110 is not near the user's face. In another example, assessment system 100 may select the audio data from the capture device based on the intensity of the audio data (e.g., decibel level, etc.). For example, even where a first capture device, such as capture device 110 (e.g. smartphone), may be closer than a second capture device, such as capture device 210 (e.g., tablet), assessment system 100 may select the second/farther capture device where the intensity of the audio data collected by the second/farther capture device is greater than the intensity of the audio data collected by the first/closer capture device (e.g., degraded or distorted signal due to an obstructed microphone, etc.). In another example, assessment system 100 may select the capture device based on the type of audio data captured. For example, where the captured audio data is speech-based (e.g., call and response from a personal trainer, spontaneous conversation, etc.), assessment system 100 may select any of the one or more devices that capture the audio data. However, where the captured audio is breath-based (e.g., yoga, meditation, etc.), assessment system 100 may select the audio data from the one or more devices that are head-worn devices (e.g., mixed-reality headset, earbuds, etc.), which may be better suited to detect the subtle changes between inhales and exhales of the breath-based audio. In another aspect, assessment system 100 may select multiple capture devices (e.g., secondary capture device, etc.) from the one or more devices to collect data associated with the user. In one example, assessment system 100 may switch between multiple capture devices over the duration of a particular activity. For example, a user may start a conversation with a first capture device, such as capture device 510 (e.g., earbuds), but end the conversation with a second capture device, such as capture device 110 (e.g., smartphone), such as when the first capture device becomes disengaged, loses battery capacity, etc. In such instances, assessment system 100 may select the audio data from both the first capture device to capture a first segment of the audio data and the second capture device to capture a second segment of the audio data. In this way, assessment system 100 may "stitch together" audio data from multiple devices to capture audio data for the duration of a particular activity. It should be noted that for clarity and conciseness not all of the one or more devices have been described in the examples above, and that the examples provided are illustrative, not exhaustive, of the operations performed by assessment system 100 where other variations are contemplated.

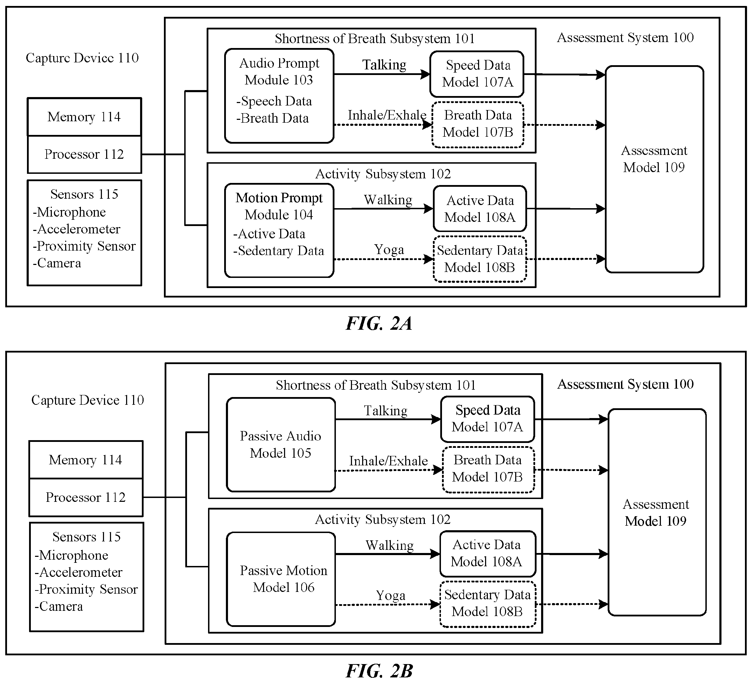

Referring now to FIGS. 2A-2D, a schematic illustration of assessment system 100 is shown on capture device 110. It has been observed that health and fitness assessments may include an analysis of overlapping or concurrent data sets where, for example, such assessments may analyze the changes in a speech pattern of a user before, during and after an activity (e.g., talk test, etc.). As such, in aspects, assessment system 100 may include shortness of breath subsystem 101 to analyze audio data collected by the one or more devices, activity subsystem 102 to analyze motion data collected by the one or more devices, and assessment model 109 to combine data from shortness of breath subsystem 101 and activity subsystem 102 to ultimately provide an overall health or fitness assessment for the user.

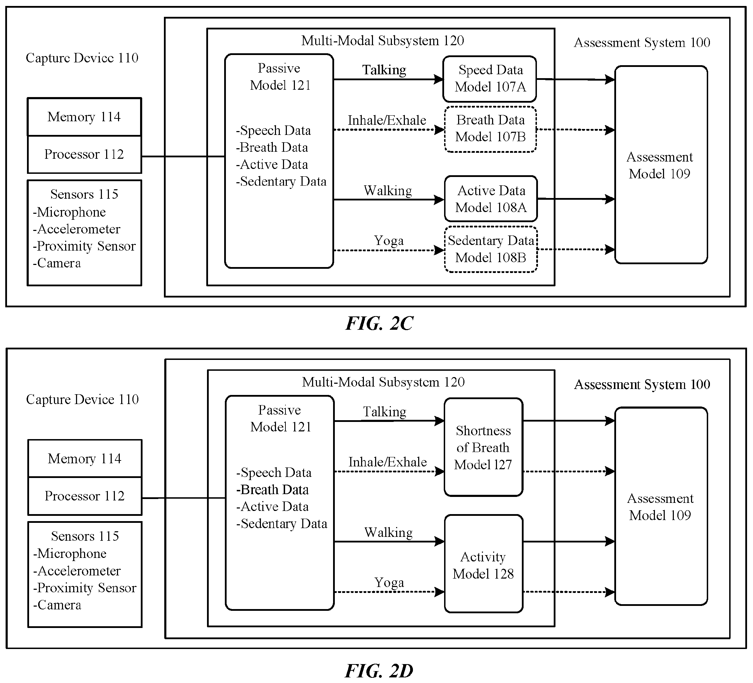

In aspects, the machine learning models of assessment system 100 may be segmented or compartmentalized to varying degrees. In some aspects, the machine learning models may be highly segmented or compartmentalized. For example, as illustrated in FIGS. 2A-2B, assessment system 100 includes individual machine learning models for passive audio data (e.g., passive audio model 105), passive motion data (e.g., passive motion model 106), different types of audio data (e.g., models 107A and 107B), and different types of motion data (e.g., models 108A and 108B). In such instances, assessment system 100 may include different machine learning models, such as convolutional neural networks, recurrent neural networks (e.g., long short-term memory ("LSTM"), etc., where the different machine learning modules may perform different operations. For example, in one aspect, the machine learning models utilized by shortness of breath subsystem (e.g., models107A and 107B) and activity subsystem (e.g., models 108A and 108B) may implement convolutional neural network models, whereas the machine learning model utilized by assessment model 109 may implement LSTM models. It should be noted that such variations in the degree of segmentation or compartmentalization of the machine learning models as well as the different types of machine learning models are not exhaustive and that other variations are contemplated. In other aspects, shortness of breath subsystem 101 and activity subsystem 102 may be collapsed into the same subsystem so rather than smaller models that analyze audio and motion data separately, a larger singular model would perform such operations together. For example, as illustrated in the example of FIG. 2C, assessment system 100 may include multi-modal subsystem 120 that includes passive model 121 to receive and analyze multi-modal data (e.g., audio data, motion data, vital sign data, etc.), which may then be further analyzed by a particular machine learning model trained on a particular data set, such as model 107A trained on speech data, model 107B trained on breath data, etc. In another example, as illustrated in the example of FIG. 2D, assessment system 100 may include multi-modal subsystem 120 that includes passive model 121 to receive and analyze multi-modal data (e.g., audio data, motion data, vital sign data, etc.), which may then be analyzed by a machine learning model trained on a broader set of data, such as shortness of breath model 127 trained on both speech data and breath data, activity model 128 trained on active data and sedentary data, etc. In the interest of clarity and conciseness, the segmented or compartmentalized aspects illustrated in FIGS. 2A-2B are discussed below in order to aid in differentiating between the various operations and functions performed by assessment system 100 although it is contemplated that all or some of such operations and functions may be performed in a combined or multi-modal model similar to the examples of FIGS. 2C-2D.

In aspects, the manner in which assessment system 100 collects data associated with a user (e.g., audio data, motion data, physiological data, environmental data, etc.) from the one or more devices (e.g., capture devices, companion devices, etc.) may vary. For example, in FIG. 2A, assessment system 100 includes audio prompt module 103 and motion prompt module 104 to collect data associated with the user in response to a prompt by the user through the one or more devices, whereas FIG. 2B includes a passive audio model 105 and passive motion model 106 to collect data passively in the absence of a prompt by the user through the one or more devices. In this way, data may be collected "actively" or deliberately in that the data collection may be user-initiated (FIG. 2A), as well as "passively" or incidentally/opportunistically in that the data collection may be system-initiated based on user context (FIG. 2B).

In some aspects, assessment system 100 may actively collect data associated with the user (e.g., audio data, motion data, physiological data, environmental data, etc.). In reference to FIG. 2A, the data collected by shortness of breath subsystem 101 and activity subsystem 102 may be deliberately provided by the user where, for example, a user accesses a particular software application (e.g., health application, fitness application, etc.) or responds to a query by a digital assistant, etc. In such instances, the user may provide input related to a specific audio-related activity (e.g., talk test, 20-minute meditation session, etc.) and/or a specific motion-related activity (e.g., 20-minute treadmill session, etc.). Based on the input received by audio prompt module 103, assessment system 100 may then "call up" the appropriate machine learning model trained on data that corresponds to the input provided by the user. Similarly, based on the audio-related input received by motion prompt module 104, assessment system 100 may call up the appropriate machine learning model trained on data that corresponds to the motion-related input provided by the user.

In other aspects, assessment system 100 may passively collect data associated with the user (e.g., audio data, motion data, physiological data, environmental data, etc.). For example, the data collected by shortness of breath subsystem 101 and activity subsystem 102 may be collected passively in the absence of a prompt by the user and then analyzed by a passive audio machine learning model and a passive motion machine learning model. In this way, assessment system 100 may determine a user context (e.g., walking, receiving a phone call, etc.) based on the passively collected data associated with the user, where the user context may initiate the speech pattern and/or motion data analyses. For example, in reference to FIG. 2B, passive audio model 105 and passive motion model 106 may collect data when, for example, a user may be walking and then simultaneously receives a phone call in which they engage in a conversation. In such instances, where a confluence of audio data and motion data may occur without deliberate notice to assessment system 100, passive audio model 105 may analyze such data to determine the type of audio data detected from the user (e.g., speech pattern, breath, etc.). Similarly, passive motion model 106 may analyze such data to determine the type of motion data detected from the user (e.g., walking, running, etc.). The active and passive collection of data not only applies to audio data and motion data as described above, but also to other data collected by the one or more sensors of the one or more devices. In one example, physiological data may be collected actively or passively by physiological sensors (e.g., photoplethysmography (PPG) sensors, electrocardiogram (ECG) sensors, electrocardiogram (EKG) sensors, bioimpedance sensors, etc.). In another example, environmental data may be collected actively or passively by environmental sensors (e.g., particulate matter (PM) sensors, carbon monoxide (CO) sensors, etc.). It should be noted that these examples are merely illustrative, not exhaustive, and that other examples of active and passive data collection of data associated with the user are contemplated.

Based on the data collected by the one or more devices either in response to a prompt as described in FIG. 2A, collected passively as described in FIG. 2B or some combination thereof, assessment system 100 may then apply a particular machine learning model to the data to generate both shortness of breath metrics and activity metrics. A shortness of breath metric may include a classification (e.g., short of breath, breathless, etc.), level (e.g., "high" for labored speech, etc.), score/grade (e.g., 5 for a strained speech pattern, 10 for a gasping speech pattern, etc.), etc. for the audio data over the duration of a particular activity, where the shortness of breath metric may vary over the duration of the particular activity. Similarly, an activity metric may include a classification (e.g., sedentary activity, strenuous activity, etc.), level (e.g., "high" for maximal effort, etc.) or score/grade (e.g., 1 for at rest, 3 for light activity, etc.), etc. for the motion data over the duration of a particular activity, where the activity metric may vary over the duration of the particular activity. In some aspects, in addition to audio data (e.g., speech data, breath data, etc.) and activity data (e.g., active data, sedentary data, etc.), the shortness of breath and/or activity metrics may be informed by or supplemented by data related to the vital signs of the user. For example, data related to the vital signs of the user may include changes in heart rate (e.g., electrocardiogram), changes in blood volume of the tissue of the user (e.g., photoplethysmogram), etc., where such data may be derived from or detected by the sensors of the one or more devices, in particular the wearable devices worn by a user (e.g., smartwatch, earbuds, etc.). In such instances, the data related to the vital signs of the user may be implemented as part of a dedicated vital sign module (similar to the segmented/compartmentalized examples of FIGS. 2A-2B) or as an additional mode in a multi-modal system where such data may be combined with the other data (e.g., audio data, activity data, etc.) in a singular machine learning model (similar to the multi-modal examples of FIGS. 2C-2D).

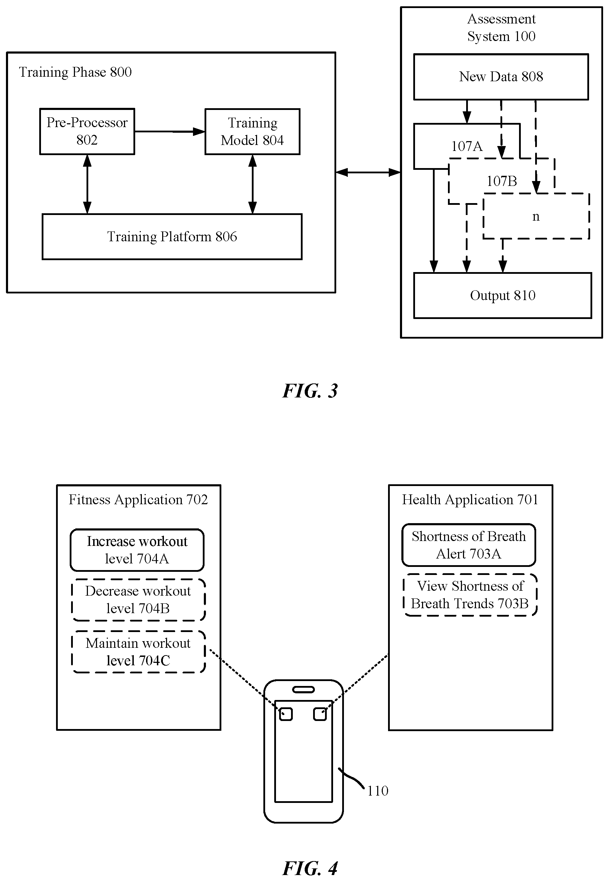

In instances where assessment system 100 utilizes a machine-learning based model, assessment system 100 can be trained to classify sounds (e.g., speech, breath, etc.), classify actions (e.g., walking, running, etc.), etc. by using one or more well-known or widely available training techniques such as supervised learning, unsupervised learning, and/or reinforcement learning techniques. For example, in reference to FIG. 3, a diagram of training phase 800 is shown in accordance with aspects. Training phase 800 may include pre-processor 802, training model 804 and training platform 806, where training platform 806 may include a suitable conventional computing device (e.g., one or more GPUs, etc.) and may be implemented as a distributed network of computing devices upon which pre-processor 802 and training model 804 may operate. For example, at pre-processor 802, training data may be provided to illustrate the features that assessment system 100 seeks to identify and quantify. Such training data may typically include a plurality of training instances, where the training instances may include a vector of values with a corresponding target (e.g., label, value, etc.). Training model 804 may be trained to map from the training data (e.g., an input vector of values) to an estimated target and to ultimately identify and quantify features within new data. Training model 804 may then be deployed to assessment system 100 and implemented as one of the machine learning models described in accordance with the aspects described. In one example, where the machine learning models of assessment system 100 may be implemented as segmented/compartmentalized models (similar to the examples of FIGS. 2A-2B), training model 804 may be trained on speech data to be implemented as model 107A, trained on breath data to be implemented as model 107B, and so on. In another example, where the machine learning models of assessment system 100 may be implemented as multi-modal models (similar to the examples of FIGS. 2C-2D), training model 804 may be trained on a combination of speech and breath, and may be implemented as shortness of breath model 127, for example. Once implemented, the machine learning models of assessment system 100 may receive new data 808, such as data from the sensors (sensors 115, sensors, 215, etc.) of the one or more devices (e.g., capture device 110, capture device 210, etc.) and then generate a corresponding output 810. It should be noted that training phase 800 may be stored on any of the one or more devices (e.g., capture device 110, capture device 210, etc.), any companion device, and/or on an external computer system (e.g., cloud network, etc.).

In aspects, shortness of breath subsystem 101 may compare the characteristics of the data collected by the one or more devices to the characteristics of the training data for each of the machine learning models. It should be noted that such models may be trained based on the type of audio data (e.g., speech pattern data, breath data, etc.), the type of motion data (e.g., walking data, sedentary data, etc.), the type of capture device (e.g., smartphone, earbuds, laptop, etc.), etc. In one example, assessment system 100 may determine that the user is walking and talking on their smartphone. In such instances, shortness of breath subsystem 101 may compare the speech pattern data from passive audio model 105 to the training data of the machine learning models stored on memory 114 of capture device 110 and then select the machine learning model 107A (specifically trained on speech pattern data collected by smartphones). Similarly, activity subsystem 102 may select machine learning model 108A (trained specifically on walking data) to analyze the walking data collected by capture device 110. In another example, assessment system 100 may determine that the user is practicing yoga with their earbuds in. In such instances, shortness of breath subsystem 101 may select machine learning model 107B (trained specifically on breath data collected by earbuds) to analyze the breath data collected by capture device 110 to generate a shortness of breath metric. Similarly, activity subsystem 102 may select machine learning model 108B (specifically trained on yoga data) to analyze the yoga data collected by capture device 110 to generate an activity metric.

Referring back to FIGS. 2A-2B, based on the shortness of breath metrics generated by shortness of breath subsystem 101 and the activity metrics generated by activity subsystem 102, assessment model 109 may determine an overall health or fitness metric (or assessment metric) for the user. It has been observed that analyzing the shortness of breath of the user in response to an activity may provide an important metric of the health, fitness, and wellness of the user (e.g., talk test, Borg scale, MRC dyspnea scale, etc.). In the example of FIGS. 2A-2B, shortness of breath subsystem 101 may send shortness of breath metrics to assessment model 109, and activity subsystem 102 may also send activity metrics to assessment model 109 where the time periods for both data sets overlap. Assessment model 109 may then analyze the overlapping audio and motion data sets to determine an overall health or fitness metric similar to the Borg scale or MRC dyspnea scale. An assessment metric may include a classification, label, score/grade, etc. where, for example, a shortness of breath metric of 8 (e.g., labored speech) and an activity metric of 10 (e.g., strenuous activity) may generate an assessment metric or grade of 1 (e.g., normal/expected). In another example, a shortness of breath metric of 9 (e.g., labored speech) and an activity metric of 3 (e.g., light activity) may generate an assessment metric or grade of 3 (e.g., abnormal/unexpected).

In one aspect, since the shortness of breath data may be analyzed over the course of an activity, assessment model 109 may include time series machine learning models (e.g., long short-term memory models, autoregressive integrated moving average models, etc.), although other types of machine learning models are contemplated. In one example, a user may have a low level of shortness of breath at the start of a walk, maintain the low level of shortness of breath after 100 steps, but then by the end of the walk the shortness of breath of the user may rise to a high level. In such instances, assessment model 109 may determine a metric for the user, where the metric may fall into a particular group for the corresponding levels of shortness of breath and activity. In this way, assessment system 100 may provide health and fitness metrics in a manner similar to what a third party may typically administer (e.g., clinician, personal trainer, etc.) but with more accurate, robust and actionable data than conventional methods may provide.

Referring now to FIG. 4, an illustration of example user interface of an application is shown in accordance with an aspect. Assessment metrics may be stored on a memory of the capture device, a memory of a companion device or an external computer system (e.g., cloud server, etc.). In addition, assessment metrics may be accessible through an application of the capture device or any of the one or more devices that are communicably linked to the capture device. In one aspect, assessment metrics may be accessible through a health application where a user may review alerts, trends, etc. Further, the user may also share such data with a healthcare professional through the health application in order to make more informed decisions regarding their medical care. In another aspect, assessment metrics may be accessible through a fitness application where a user may customize their workout based on the assessment metrics. For example, where a user engages in a moderate level workout but experiences a high level of shortness of breath, the fitness application may provide a notification to reduce the level of the workout. Conversely, where a user engages in a moderate level workout but experiences a low level of shortness of breath, the fitness application may provide a notification to raise the level of the workout. For example, as illustrated in FIG. 4, capture device 110 may include health application 701 that may display various types of notifications, such as a shortness of breath alert 703A, shortness of breath trends 703B, etc. Further, capture device 110 may also include fitness application 702 that may display various customized workout recommendations, such as increases to workout level 704A, reductions in workout level 704B or to maintain a workout level 704C, as well as other fitness-related notifications.

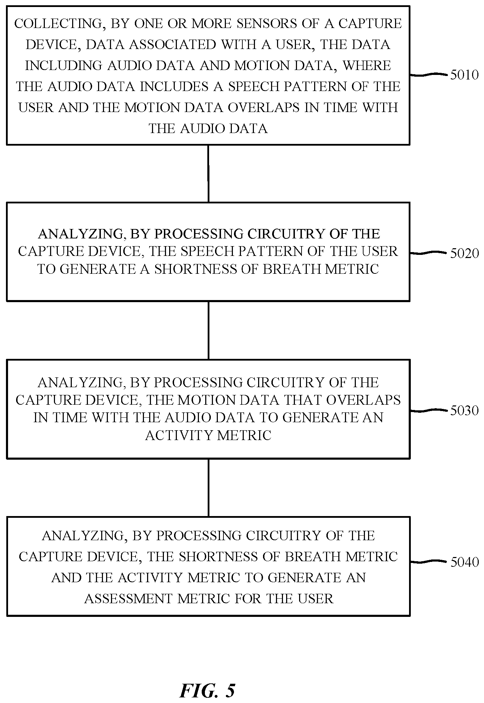

FIG. 5 is a flow chart of a method for generating a shortness of breath metric. In aspects, the method may be performed by assessment system 100, which may be located in whole or in part on any of the one or more devices. In one aspect, assessment system 100 may be located on the capture device (e.g., earbuds) that captures or collects data from the user so that the operations may be performed on-device. In another aspect, assessment system 100 may be located on a companion device (e.g., smartphone) where, for example, the capture device (e.g., earbuds) lacks the storage, memory, compute resources, or battery capacity to perform such operations. In such instances, assessment system may be located in whole on the companion device, or, in part on the capture device and in part on the companion device so that, for example, shortness of breath subsystem 101 may be located on the capture device (e.g., earbuds) and activity subsystem 102 and assessment model 109 may be located on the companion device (e.g., smartphone). In another aspect still, assessment system 100 may be located, in whole or in part, on an external computer system (e.g., physical server, virtual server, etc.) where the operations may be performed on the external computer system and then transmitted back to the one or more devices that served as the capture device(s) and/or companion device(s).

In further reference to FIG. 5, at operation 5010 a capture device may collect data associated with the user (e.g., audio data, motion data, physiological data, environmental data), where collecting data associated with the user may include acquiring signals from sensors (e.g., waveforms, accelerometer output, gyroscope signals, etc.), receiving processed data from other systems/subsystems, and obtaining information through any acquisition method. In one example, where a microphone receives audio signals, the audio signals may be converted into audio data (e.g., spectrogram, etc.) to be analyzed by a machine learning model of assessment system 100. In some aspects, the audio data may be collected by one capture device (e.g., earbuds). In other aspects, the audio data may be collected by multiple capture devices (e.g., smartphone and tablet, etc.). In such instances, assessment system 100 may select the audio from one, some or all of the capture devices. For example, assessment system 100 may select audio data from a capture device based on its proximity to the user (e.g., closest device), the intensity of the audio data (e.g., decibel level, etc.), the type of audio data captured where, for example, breath-based data may be more suitably captured by head-worn devices (e.g., mixed-reality headset, earbuds, etc.), etc. Further still, assessment system 100 may "stitch together" audio from multiple capture devices where, for example, a user starts an activity on a first capture device (e.g., smartphone, etc.) but ends the activity on a second capture device (e.g., earbuds, etc.). In addition, the capture device may collect motion data from any of the one or more devices, where such motion data overlaps in time with the audio data.

In further reference to FIG. 5, at operation 5020 assessment system 100 may analyze the audio data of the user to determine a shortness of breath metric. In some aspects, the analysis of the audio data may be performed by a first machine learning model (e.g., model 107A, model 107B, etc.). The audio data may include speech pattern data (e.g., call and response from a virtual trainer, spontaneous conversation, etc.), breathing data (e.g., inhale, exhale, etc.) as well as other types of respiratory data. Further, at operation 5030 assessment system 100 may analyze the motion data of the user to determine an activity metric. In some aspects, the analysis of the motion data may be performed by a second machine learning model (e.g., model 108A, model 108B, etc.). The motion data may include active motion data (e.g., walking, running, biking, etc.) as well as inactive or sedentary motion data (e.g., sitting at a desk, meditating, etc.) detected by the one or more sensors of the one or more devices. In some instances, assessment system 100 may actively collect such audio and motion data as the result of a prompt from a digital assistant and/or an input provided by the user within an application (e.g., health application, fitness application, etc.). For example, before starting a workout, a user may open a health application and either perform the first segment of a talk test and/or provide permissions that all audio data may be captured for the duration of the workout for the purpose of measuring the described health and fitness metrics, where the type of workout may also be specified. In other instances, such as when the audio data or motion data do not result from a prompt from a digital assistant and/or an input provided by the user within an application, for example, assessment system 100 may passively collect audio data and motion data for the purpose of measuring the described health and fitness metrics. In one aspect, assessment system 100 may utilize a third machine learning model (e.g., passive audio model 105, etc.) and a fourth machine learning model (e.g., passive motion model 106, etc.) to analyze the audio and motion data, respectively, in order to determine the type of audio data and motion data collected or captured by the one or more devices. In this way, assessment system 100 may still measure the described health and fitness metrics in spontaneous moments, such as walking and receiving a phone call, without the need for any deliberate action on the part of the user.

It should be noted that the machine learning models described at operations 5020 and 5030 have been compartmentalized with regard to audio data (e.g., model 107A, model 107B, etc.), motion data (e.g., model 108A, 108B, etc.), and passive data (e.g., passive audio model 105, passive motion model 106, etc.), as well as for the overall health and fitness metric (e.g., assessment model 109), as described in the examples of FIGS. 2A-2B. In other aspects, such machine learning models may be consolidated to varying degrees, as described in the examples of FIGS. 2C-2D. In one example, all such machine learning models may be consolidated or combined into a single machine learning module. In another example, passive audio model 105 and passive motion model 106 may be combined into a single machine learning model dedicated to analyzing all passive data (e.g., passive model 121, etc.). In another example, machine learning model 107A for analyzing speech pattern data and machine learning model 107B for analyzing breath data may be combined into a single machine learning model dedicated to analyzing all audio data (e.g., shortness of breath model 127, etc.). Similarly, machine learning model 108A and machine learning model 108B may be combined into a single machine learning model dedicated to analyzing all motion data (e.g., activity model 128, etc.). These variations described are not exhaustive and are only representative of the different types of machine learning models (e.g., CNN, LSTM, etc.) and different combinations of machine learning models contemplated.

Based on the shortness of breath metrics provided by shortness of breath subsystem 101 and the activity metrics provided by activity subsystem 102, assessment model 109 may determine an overall health or fitness metric for a user at operation 5040. Such metrics may then be stored on a memory of the capture device (e.g., memory 114 of capture device 110, etc.), companion device or an external computer system where they may be retrieved by any of the one or more devices communicably linked to each other and/or the external computer system. In some aspects, such metrics may be accessed by the user through an application (e.g., health application, fitness application, etc.). In one example, the user may open a health application to review archived shortness of breath metrics, view trends, etc. In another example, the user may open a fitness application to review archived shortness of breath metrics related to a particular workout or activity, receive workout recommendations based on the shortness of breath metrics, etc.

As described, one aspect of the present technology is the gathering and use of data available from specific and legitimate sources to provide frequent and accurate health assessments based on shortness of breath. It is contemplated that, in some instances, this gathered data may include personal information data that uniquely identifies or can be used to identify a specific person. Such personal information data can include demographic data, location-based data, online identifiers, telephone numbers, email addresses, home addresses, data or records relating to a user's health or level of fitness (e.g., vital signs measurements, medication information, exercise information, etc.), date of birth, or any other personal information. It is recognized that the use of such personal information data, in the present technology, can be used to the benefit of users. In some instances, health and fitness data may be used, in accordance with the user's preferences to provide insights into their general wellness or may be used as positive feedback to individuals using technology to pursue wellness goals based on shortness of breath metrics, for example.

It is contemplated that those entities responsible for the collection, analysis, disclosure, transfer, storage, or other use of such personal information data will comply with well-established privacy policies and/or privacy practices. In particular, such entities would be expected to implement and consistently apply privacy practices that are generally recognized as meeting or exceeding industry or governmental requirements for maintaining the privacy of users. Such information regarding the use of personal data should be prominent and easily accessible by users and should be updated as the collection and/or use of data changes. Personal information from users should be collected for legitimate uses only. Further, such collection/sharing should occur only after receiving the consent of the users or other legitimate basis specified in applicable law. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and adapted to applicable laws and standards, including jurisdiction-specific considerations that may serve to impose a higher standard. For instance, in the U.S., collection of or access to certain health data may be governed by federal and/or state laws, such as the Health Insurance Portability and Accountability Act (HIPAA); whereas health data in other countries may be subject to other regulations and policies and should be handled accordingly.

Despite the foregoing, it is also contemplated that aspects in which users selectively block the use of, or access to, personal information data. That is, the present disclosure contemplates that hardware and/or software elements can be provided to prevent or block access to such personal information data. For example, such as in the case with health and fitness data, the present technology can be configured to allow users to select to "opt in" or "opt out" of participation in the collection of personal information data during registration for services or anytime thereafter. In addition to providing "opt in" and "opt out" options, it is contemplated that providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing identifiers, controlling the amount or specificity of data stored (e.g., collecting location data at city level rather than at an address level), controlling how data is stored (e.g., aggregating data across users), and/or other methods such as differential privacy.

Therefore, although the present disclosure broadly covers use of personal information data to implement one or more various disclosed aspects, the present disclosure also contemplates that the various aspects can also be implemented without the need for accessing such personal information data. That is, the various aspects of the present technology are not rendered inoperable due to the lack of all or a portion of such personal information data. For example, content can be selected and delivered to users based on aggregated non-personal information data or a bare minimum amount of personal information, such as the content being handled only on the user's device or other non-personal information available to the content delivery services.

In utilizing the various aspects of the aspects, it would become apparent to one skilled in the art that combinations or variations of the above aspects are possible for generating a speech-based shortness of breath metric. Although the aspects have been described in language specific to structural features and/or methodological acts, it is to be understood that the appended claims are not necessarily limited to the specific features or acts described. The specific features and acts disclosed are instead to be understood as aspects of the claims useful for illustration.

### NUMBERED STATEMENTS OF INVENTION

1. A speech-based system comprising:
   one or more devices, wherein the one or more devices include a capture device, the capture device including:
   processing circuitry; and
   a memory coupled to the processing circuitry, the memory storing instructions that, when executed by the processing circuitry, cause the system to perform operations that include:
      collecting, by one or more sensors of the capture device, data associated with a user, the data including audio data and motion data, wherein the audio data includes a speech pattern of the user and the motion data overlaps in time with the audio data;
      analyzing the speech pattern of the user to generate a shortness of breath metric;
      analyzing the motion data that overlaps in time with the audio data to generate an activity metric; and
      analyzing the shortness of breath metric and the activity metric to generate an assessment metric for the user.
2. The speech-based system of statement 1, wherein the one or more devices include a companion device, the companion device including:
   processing circuitry; and
   a memory coupled to the processing circuitry, the memory storing instructions that, where executed by the processing circuitry, causes the system to perform operations that include:
      collecting, by one or more sensors of the companion device, data associated with the user, the data including audio data and motion data, wherein the audio data includes the speech pattern of the user and the motion data overlaps in time with the audio data;
      analyzing the speech pattern of the user to generate the shortness of breath metric; and
      analyzing the motion data that overlaps in time with the audio data to generate the activity metric;
      wherein the companion device and the capture device are communicatively linked to share the data associated with the user.
3. The speech-based system of statement 2, wherein collecting data associated with the user further includes physiological data and environmental data.
4. The speech-based system of statement 2, wherein collecting the data associated with the user occurs actively in response to a prompt provided by the user.
5. The speech-based system of statement 2, wherein collecting the data associated with the user occurs passively to determine a user context, the user context initiating the speech pattern and motion data analyses.
6. The speech-based system of statement 1, when multiple devices of the one or more devices capture the audio data, selecting the audio data from one of the multiple devices based on one or more characteristics of the audio data, the one or more characteristics including an intensity of the audio data or a type of audio data.
7. The speech-based system of statement 1, when multiple devices of the one or more devices capture the audio data, selecting a first device to capture a first audio segment and a second device to capture a second audio segment of the audio data.
8. The speech-based system of statement 1, wherein the assessment metric may include shortness of breath trends of the user.
9. A speech-based method comprising:
   collecting, by one or more sensors of a capture device, data associated with a user, the data including audio data and motion data, wherein the audio data includes a speech pattern of the user and the motion data overlaps in time with the audio data;
   analyzing, by processing circuitry of the capture device, the speech pattern of the user to generate a shortness of breath metric;
   analyzing, by processing circuitry of the capture device, the motion data that overlaps in time with the audio data to generate an activity metric; and
   analyzing, by processing circuitry of the capture device, the shortness of breath metric and the activity metric to generate an assessment metric for the user.
10. The speech-based method of statement 9, further including:
   collecting, by one or more sensors of a companion device, data associated with the user, the data including audio data and motion data, wherein the audio data includes the speech pattern of the user and the motion data overlaps in time with the audio data;
   analyzing, by processing circuitry of the companion device, the speech pattern of the user to generate a shortness of breath metric; and
   analyzing, by processing circuitry of the companion device, the motion data that overlaps in time with the audio data to generate the activity metric;
   wherein the companion device and the capture device are communicatively linked to share the data associated with the user.
11. The speech-based method of statement 10, wherein collecting data associated with the user further includes physiological data and environmental data.
12. The speech-based method of statement 10, wherein collecting the data associated with the user occurs actively in response to a prompt provided by the user.
13. The speech-based method of statement 10, wherein collecting the data associated with the user occur passively to determine a user context, the user context initiating the speech pattern and motion data analyses.
14. The speech-based method of statement 9, when multiple devices capture the audio data, selecting the audio data based on one or more characteristics of the audio data, the one or more characteristics including an intensity of the audio data or a type of audio data.
15. The speech-based method of statement 9, when multiple devices capture the audio data, selecting a first device to capture a first audio segment and a second device to capture a second audio segment of the audio data.
16. The speech-based method of statement 9, wherein the assessment metric may include shortness of breath trends of the user.
17. A non-transitory computer readable medium to store instructions that, when executed by processing circuitry of a speech-based computer system, cause the speech-based computer system to:
   collect, by one or more sensors of a capture device, data associated with a user, the data including audio data and motion data, wherein the audio data includes a speech pattern of the user and the motion data overlaps in time with the audio data;
   analyze, by processing circuitry of the capture device, the speech pattern of the user to generate a shortness of breath metric;
   analyze, by processing circuitry of the capture device, the motion data that overlaps in time with the audio data to generate an activity metric; and
   analyze, by processing circuitry of the capture device, the shortness of breath metric and the activity metric to generate an assessment metric for the user.
18. The non-transitory computer readable medium of statement 17, wherein the processing circuitry executes instructions to cause the speech-based system to:
   collect, by one or more sensors of a companion device, data associated with the user, the data including audio data and motion data, wherein the audio data includes the speech pattern of the user and the motion data overlaps in time with the audio data;
   analyze, by processing circuitry of the companion device, the speech pattern of the user to generate a shortness of breath metric; and
   analyze, by processing circuitry of the companion device, the motion data that overlaps in time with the audio data to generate the activity metric;
   wherein the companion device and the capture device are communicatively linked to share the data associated with the user.
19. The non-transitory computer readable medium of statement 18, wherein the processing circuitry executes instructions to cause the speech-based system to collect the data associated with the user actively in response to a prompt provided by the user.
20. The non-transitory computer readable medium of statement 18, wherein the processing circuitry executes instructions to cause the speech-based system to collect the data associated with the user passively to determine a user context, the user context initiating the speech pattern and motion analyses.

## Claims

1. A speech-based system comprising:
one or more devices, wherein the one or more devices include a capture device, the capture device including:
processing circuitry; and
a memory coupled to the processing circuitry, the memory storing instructions that, when executed by the processing circuitry, cause the system to perform operations that include:
collecting, by one or more sensors of the capture device, data associated with a user, the data including audio data and motion data, wherein the audio data includes a speech pattern of the user and the motion data overlaps in time with the audio data;
analyzing the speech pattern of the user to generate a shortness of breath metric;
analyzing the motion data that overlaps in time with the audio data to generate an activity metric; and
analyzing the shortness of breath metric and the activity metric to generate an assessment metric for the user.

2. The speech-based system of claim 1, wherein the one or more devices include a companion device, the companion device including:
processing circuitry; and
a memory coupled to the processing circuitry, the memory storing instructions that, where executed by the processing circuitry, causes the system to perform operations that include:
collecting, by one or more sensors of the companion device, data associated with the user, the data including audio data and motion data, wherein the audio data includes the speech pattern of the user and the motion data overlaps in time with the audio data;
analyzing the speech pattern of the user to generate the shortness of breath metric; and
analyzing the motion data that overlaps in time with the audio data to generate the activity metric;
wherein the companion device and the capture device are communicatively linked to share the data associated with the user.

3. The speech-based system of claim 2, wherein collecting data associated with the user further includes physiological data and environmental data.

4. The speech-based system of claim 2 or 3, wherein collecting the data associated with the user occurs actively in response to a prompt provided by the user.

5. The speech-based system of claim 2 or 3, wherein collecting the data associated with the user occurs passively to determine a user context, the user context initiating the speech pattern and motion data analyses.

6. The speech-based system of any preceding claim, when multiple devices of the one or more devices capture the audio data, selecting the audio data from one of the multiple devices based on one or more characteristics of the audio data, the one or more characteristics including an intensity of the audio data or a type of audio data.

7. The speech-based system of any preceding claim, when multiple devices of the one or more devices capture the audio data, selecting a first device to capture a first audio segment and a second device to capture a second audio segment of the audio data.

8. The speech-based system of any preceding claim, wherein the assessment metric may include shortness of breath trends of the user.

9. A speech-based method comprising:
collecting, by one or more sensors of a capture device, data associated with a user, the data including audio data and motion data, wherein the audio data includes a speech pattern of the user and the motion data overlaps in time with the audio data;
analyzing, by processing circuitry of the capture device, the speech pattern of the user to generate a shortness of breath metric;
analyzing, by processing circuitry of the capture device, the motion data that overlaps in time with the audio data to generate an activity metric; and
analyzing, by processing circuitry of the capture device, the shortness of breath metric and the activity metric to generate an assessment metric for the user.

10. The speech-based method of claim 9, further including:
collecting, by one or more sensors of a companion device, data associated with the user, the data including audio data and motion data, wherein the audio data includes the speech pattern of the user and the motion data overlaps in time with the audio data;
analyzing, by processing circuitry of the companion device, the speech pattern of the user to generate a shortness of breath metric; and
analyzing, by processing circuitry of the companion device, the motion data that overlaps in time with the audio data to generate the activity metric;
wherein the companion device and the capture device are communicatively linked to share the data associated with the user.

11. The speech-based method of claim 10, wherein collecting data associated with the user further includes physiological data and environmental data.

12. The speech-based method of claim 10 or 11, wherein collecting the data associated with the user occurs actively in response to a prompt provided by the user, or wherein collecting the data associated with the user occur passively to determine a user context, the user context initiating the speech pattern and motion data analyses.

13. The speech-based method of any of claims 9 to 12, when multiple devices capture the audio data, selecting the audio data based on one or more characteristics of the audio data, the one or more characteristics including an intensity of the audio data or a type of audio data, or when multiple devices capture the audio data, selecting a first device to capture a first audio segment and a second device to capture a second audio segment of the audio data.

14. The speech-based method of any of claims 9 to 13, wherein the assessment metric may include shortness of breath trends of the user.

15. A computer readable medium comprising instructions that, when executed by processing circuitry of a speech-based computer system, cause the speech-based computer system to:
perform the method of any of claims 9 to 14.
